# EUROPEAN PATENT SPECIFICATION

(11) **EP 2 861 233 B1**
(45) Date of publication and mention of the grant of the patent: **06.11.2019**
(21) Application number: 13807463.8
(22) Date of filing: 18.06.2013
(51) Int. Cl.: A61K 9/70, A61K 31/57, A61K 31/565, A61K 47/14, A61K 45/06

(54) **TRANSDERMAL HORMONE REPLACEMENT THERAPIES**
TRANSDERMALE HORMONERSATZTHERAPIEN
TRAITEMENT TRANSDERMIQUE DE SUBSTITUTION HORMONALE

(30) Priority: 18.06.2012 US 201261661302 P; 20.06.2012 US 201261662265 P; 21.11.2012 US 201213684002; 25.01.2013 WO PCT/US2013/023309; 15.03.2013 US 201313843362
(43) Date of publication of application: 22.04.2015
(73) Proprietor: TherapeuticsMD, Inc., Boca Raton, FL 33431 (US)
(72) Inventor: BERNICK, Brian A., Boca Raton, Florida 33496 (US); AMADIO, Julia M., Boca Raton, FL 33486 (US); PERSICANER, Peter H.R., Boca Raton, Florida 33496 (US); CACACE, Janice Louise, St Petersburg, FL 33702 (US); IRANI, Neda, Palm Beach Gardens, Florida 33410 (US)
(74) Representative: Murgitroyd & Company
(86) International application number: PCT/US2013/046444
(87) International publication number: WO 2013/192250

(56) References cited:
- EP-A2- 0 279 977
- WO-A1-00/50007
- WO-A1-01/87276
- WO-A1-90/11064
- WO-A1-97/43989
- US-A- 4 384 096
- US-A1- 2003 077 297
- US-A1- 2005 042 268
- US-A1- 2006 134 188
- US-A1- 2007 264 349
- US-B1- 6 294 192

## Description

### FIELD

This disclosure relates to natural estrogen and progesterone replacement therapies, with transdermal formulations provided for each estrogen and progesterone alone and in combination for the treatment of pre-menopausal, peri-menopausal, menopausal and post-menopausal females in relation to the treatment of estrogen- and progesterone-deficient states, each as herein defined below.

### BACKGROUND

Hormone replacement therapy (HRT) is a medical treatment that involves the use of one or more of a group of medications designed to increase hormone levels in women who lack adequate hormone production. HRT can mitigate and prevent symptoms caused by diminished circulating estrogen and progesterone hormones regardless as to whether the subject is pre-menopausal, peri-menopausal, menopausal or post-menopausal. However, specific symptomatic states can exist during each stage of menopausal progression.

HRT via transdermal delivery is presently available in various forms. One therapy involves administration of one or more estrogens. Another involves administration of a chemical analogue of naturally occurring progesterone, called a progestin. Progesterone administration acts, in addition to treating other disease states, to mitigate certain undesirable side effects from estrogen administration including, for example, endometrial hyperplasia (thickening), and reducing the incidence of endometrial cancer.

Reference to progesterone, both naturally-occurring and bio-identical, is generally collectively referred to herein as "progesterone."

Various references generally suggest the use of the progestogen class of hormones for inclusion in transdermal therapies, typically in combination with an estrogen. The teachings within such references are generally limited to progestins however. Progestins are sometimes confused in the literature with progesterone or progestogens generally. Yet progestins are a distinct subclass of progestogens; a synthetic progestogen that has progestational effects similar to progesterone. Typically, synthetic progestins have higher activity and are administered at significantly lower dosages relative to natural progesterone.

The term "progestogen(s)," as used herein, is inclusive of naturally-occurring and bio-identical progesterone and all synthetic progestins. The term "bio-identical" hormones, as used herein,, refers to hormones which are identical in chemical structure to the hormones naturally produced by mammals, including humans, and can be used, and are often referred to as, natural hormone replacement therapy, or "NHRT."

Prevailing transdermal patch technologies utilize progestins over progesterone. This is due to, among others, the difficulty with solubilizing natural progesterone which is relatively insoluble, particularly at high dosages. Solubilized drug substances are preferred in the manufacture of the various patch technologies, particularly matrix and reservoir technologies. Synthetic progestins typically have greater potency and therefore lower dosage requirements. The reduced amount of progestin drug substance relative to a given volume of drug product generally affords relatively easier formulation solubility. For example, a drug load of 2.7 mg of northethindrone acetate is provided within a 9 square cm round COMBIPATCH (estradiol/norethindrone acetate transdermal system) product; a drug load of 4.8 mg of norethindrone acetate within the 16 square cm patch product.

Progesterone, on the other hand, is defined by a lower potency and therefore a higher dosage requirement. A much larger amount of progesterone drug substance is required, up to 200 mg, in a patch product. Given the higher dosage requirements and relative insolubility of progesterone, no transdermal patch employing a natural progesterone drug substance has been successfully commercialized.

Considering the current trend towards natural and bio-identical products, particularly with human hormones, the present disclosure provides, among other aspects, solubilized progesterone that can be used with transdermal patch technologies, and transdermal patches comprising progesterone, alone or in combination with at least one estrogen drug substance including, without limitation, estradiol, and other drug substances having estradiol or estradiol-like biological activity that are well known in the art.

Timing for HRT dosage administration is often varied cyclically, with estrogens taken daily and progesterone taken for approximately two weeks of every month; a method often referred to as "cyclic-sequential" or "sequentially-combined" HRT. This method is intended to mimic the natural menstrual cycle and typically causes menstruation similar to a period after the progesterone is stopped. This regime is most typically used in peri-menopausal or newly menopausal women as the alternative continuous method often resulting in irregular bleeding. An alternate method involving a constant dosage with both estrogen and progesterone delivered daily is called "continuous-combined" HRT. This method usually results in no menstruation and is used most often after a woman has been menopausal for some time.

With each of the cyclic-sequential or continuous combined methods, each of an estrogen and progesterone, as used herein, may be administered in the same or different dosage forms. When used in the same dosage form, the present disclosure includes each of an estrogen and progesterone in a transdermal patch. When administered in different dosage forms, the present disclosure provides for progesterone administered in a transdermal patch.

There is also a need for a pre-packaged cyclic-HRT with combination estrogen and progesterone. By way of example, doctors desiring to prescribe cyclic-HRT are required to prescribe two separate products, namely, an estrogen patch and a combination estrogen/progesterone patch or an estrogen patch along with another form of progesterone including oral or topical. Such separate dosing regimes often result in poor patient compliance due to, among others, confusion as to how and when to take each of the medications throughout the prescribed therapy period. Increased patient morbidity may result.

### SUMMARY

According to various embodiments of the disclosure, hormone replacement therapies are provided comprising solubilized progesterone alone and optionally with an estrogen, cyclic/sequential and continuous-combined dosing regimes, and administered via transdermal HRT delivery systems. When solubilized progesterone is delivered alone via a transdermal patch, one or more estrogen compounds may be delivered concurrently on the same transdermal patch, or via any other known method of delivery. In such formulations, the progesterone may be solubilized in medium chain fatty acid glycol esters, e.g., C6-C12, C6-C10, C8-C12, or C8-C10 fatty acid esters of glycerol, polyethylene glycol or propylene glycol, or mixtures thereof. Such formulations may further comprises a surfactant, such as a non-ionic surfactant, such as fatty acid esters of polyethylene glycol. As noted, such formulations may comprise estradiol.

In other embodiments, the transdermal pharmaceutical formulation of the invention may comprise formulations in which the progesterone is micronized and partially solubilized. Such formulations may also comprise fatty acid glycol esters and surfactants as briefly described above and as described in more detail below.

A 28-day or monthly regime of transdermal patches can be packaged in a single package having delivery days identified to improve and optimize compliance. Various examples of such HRT transdermal delivery systems and uses thereof, each in accordance with the present invention, are set forth below.

### BRIEF DESCRIPTION OF THE DRAWINGS

The accompanying drawings are included to provide a further understanding of the disclosure and are incorporated in and constitute a part of this specification, to illustrate embodiments of the disclosure and, together with the description, serve to explain the principles of the disclosure.
Fig. 1 illustrates a cross-sectional view of an embodiment of a transdermal patch in accordance with the present disclosure;
Fig. 2 illustrates a cross-sectional view of another embodiment of a transdermal patch in accordance with the present disclosure;
Fig. 3 illustrates a cross-sectional view of an embodiment of a transdermal reservoir in accordance with the present disclosure; and
Figs. 4, 5, 6, and 7 each illustrates a different sequence control device for a cyclic/sequential hormone replacement therapy regime in accordance with the present disclosure.

### DETAILED DESCRIPTION OF THE ILLUSTRATED EMBODIMENTS

### DEFINITIONS

The term "excipients," as used herein, refers to non-active pharmaceutical ingredients such as carriers, solvents, oils, lubricants and others used in formulating pharmaceutical products. They are generally safe for administering to mammals, including humans, according to established governmental standards, including those promulgated by the United States Food and Drug Administration.

The term "medium chain," as used herein means any medium chain carbon-containing substance, including C4-C18, and including C6-C12 substances, fatty acid esters of glycerol, fatty acids, and mono-, di-, and tri-glycerides of such substances. For further illustration, C6-C14 fatty acids, C6-C12 fatty acids, and C8-C10 fatty acids are all medium chain fatty acids and may be used in instances in which this specification calls for use of medium chain fatty acids, e.g., medium chain fatty acid esters of glycerol or other glycols.

The term "micronized progesterone," as used herein, includes micronized progesterone having an X50 particle size value below about 15 microns and/or having an X90 particle size value below about 25 microns.

The term "micronized estradiol," as used herein, includes micronized estradiol having an X90 particle size value below about 7 microns.

Unless otherwise specified, "natural," as used herein with reference to certain hormones discussed herein, means bio-identical hormones prepared to match the chemical structure and effect of those that occur naturally in mammalian bodies, including humans (i.e., endogenous). An exemplary natural estrogen is estradiol (also described as 17β-estradiol and E2) and a natural progestogen is progesterone.

The term "oil" as used herein may be any pharmaceutically acceptable substance, such as an organic oil other than peanut oil, that would suspend and/or solubilize any suitable progesterone, starting material, or precursor, including micronized progesterone as described herein. More specifically, oils may include, for example and without limitation, medium chain fatty acids, generally of the group known as medium chain fatty acids consisting of at least one mono-, di-, and triglyceride, or derivatives thereof, or combinations thereof.

The term "pharmaceutically acceptable," indicates that the designated carrier, vehicle, diluent, excipient(s), and/or salts and other derivatives is generally chemically and/or physically compatible with the other ingredients comprising the formulation, and physiologically compatible with the recipient thereof.

The term "solubilizer," as used herein, means any substance or mixture of substances that may be used to solubilize or to enhance the solubility of an active pharmaceutical ingredient ("API"), such as estradiol and/or progesterone, including, for example and without limitation, appropriate pharmaceutically acceptable excipients, such as solvents, co-solvents, surfactants, emulsifiers, oils and carriers.

The term "transdermal patch," as used herein, refers to any adhesive construct suitable to adhere to the skin of a mammal, and to deliver, among one or more drug substances, physiologically effective amounts of solubilized progesterone through the skin of a mammal, including, for example and without limitation, a human. The construct may be in the form of a flexible strip, comprising the desired amount of solubilized progesterone and one or more optional additional drug substances including estrogen.

As used herein, the term "treatment", or a derivative thereof, contemplates partial or complete inhibition of the disease state when a formulation as described herein is administered prophylactically or following the onset of the disease state for which such formulation is administered. For the purposes of the present disclosure, "prophylaxis," refers to administration of the drug substance(s) to a mammal, including human, to protect the mammal from any of the disorders set forth herein, as well as others.

The term "drug load," as used herein means the amount of drug substance in a given formulation as described herein.

The term "daily dose," as used herein means the nominal transdermal delivery rate (mg per day) of a drug substance; for example delivery of a drug substance via skin of average permeability.

The term "X50," as used herein, means that one-half of the particles in a sample are smaller in diameter than a given number. For example, micronized progesterone having an X50 of 15 microns means that, for a given sample of micronized progesterone, one-half of the particles have a diameter of less than 15 microns. Similarly, the term "X90" means that ninety percent (90%) of the particles in a sample are smaller in diameter than a given number.

"Fully solubilized progesterone" as used herein means progesterone which is about 100% in solution, e.g., at least 98% in solution.

"Partially solubilized progesterone" as used herein means progesterone which is in any state of solubilization up to but not including about 100%, e.g.., up to but not including 98% progesterone in solution and greater than or equal to 2% micronized progesterone in suspension.

As used herein, unless specified, estradiol (which is also referred to in the literature as 17β-estradiol, oestradiol, or E2) includes estradiol in anhydrous and hemihydrate forms.

### DESCRIPTION

Provided herein are transdermal pharmaceutical formulations comprising solubilized progesterone (or partially solubilized progesterone) and transdermal pharmaceutical formulations comprising solubilized (or partially solubilized) progesterone and at least one estrogen compound. Generally, transdermal patches or other transdermal formulations, e.g., gels, creams, ointments, etc., are prepared by methods well known in the art, limited only by the drug load capacity of the respective technology.

For example, one common transdermal technology employs a transdermal drug delivery device that comprises a polymer matrix (or matrices) design wherein the polymer matrix can be prepared using natural polymers including, for example and without limitation, cellulose derivatives, zein, gelatin, shellac, waxes, proteins, gums and their derivatives, natural rubber, starch and the like; synthetic elastomers including, for example and without limitation, polybutadiene, polyisobutylene, hydrin rubber, polysiloxane and other silicone pressure sensitive adhesives available from Dow Corning (Elizabethtown, KY), silicone rubber, nitrile-containing compounds, acrylonitrile, butyl rubber, styrenebutadieine rubber, neoprene and the like; and/or synthetic homopolymers or copolymers including, for example and without limitation, polyvinyl alcohol, polyvinyl chloride, polyethylene, polypropylene, polyacrylate, polystyrene, polyamide, polyurea, polyvinylpyrrolidone, polymethylmethacrylate, epoxy, polyethylene/vinyl acetate, and the like. To this matrix is applied/incorporated at least one drug substance and, frequently, penetration enhancers, commonly used one or more solvents and/or one or more surfactants. An adhesive is used to affix the transdermal patch. Such adhesives can be affixed as a layer to the drug substance-containing matrix, or as one of the ingredients used to prepare the matrix. Other matrix technologies can include crystallization inhibitors and other pharmaceutically acceptable excipients. Matrix technology can also be developed into single or multi-layer patches. See, for example, U.S. Patent Nos.: 5,474,783, 5,958,446, and 6,440,454, and PCT Application Publication No. WO 97/43989, each of which are incorporated herein by reference.

Also well known in the transdermal pharmaceutical delivery art is a transdermal drug delivery device that comprises a reservoir system, characterized by the inclusion of a reservoir: a compartment containing solubilized or suspended drug substance or substances, which is separate and distinct from the release liner. Various adhesive systems are also available for reservoir-type systems. Reservoir systems are frequently the system of choice when substantial drug loads are required. See, for example, U.S. Patent Nos.: 7,387,789, 5,656,286, 8,114,434, 6,024,976, and U.S. Patent Publication Nos.: 2012/0269878, and 2012/0283671, each of which are incorporated herein by reference.

Other transdermal patch technologies include, for example and without limitation, transdermal drug delivery device that utilize iontophoresis, electroporation, ultrasound/sonophoresis, and microscopic projection (e.g., microneedles) (see, e.g., Patel, D., et al., The Pharma Innovation, Transdermal Drug Delivery System: A Review, Vol. 1, No. 4, pg. 66-75 (2012)).

Other transdermal forms include creams, ointments, gels and the like that may be applied to the skin by rubbing with one's fingers or with a flexible applicator. Such forms also include sprays, such formulations that are aerosolized upon release from a pressurized container.

Such transdermal systems as described herein can be used for the preparation of solubilized progesterone transdermal patches, with or without the addition of at least one estrogen drug substance or other compatible drug substance(s).

Other aspects of the present disclosure includes the use of formulations as described herein, wherein a therapeutically effective amount of solubilized progesterone is at least one drug substance in said formulation for the treatment of a mammal, including humans: for endometrial hyperplasia; for secondary amenorrhea; as a method of treatment for preterm birth when said mammal has a shortened cervix; and other disease states or conditions treated with supplemental progesterone (collectively, "progesterone-deficient states"); and wherein an estrogen drug substance is optionally included in said formulations.

Additional aspects of the present disclosure include the use of formulations as described herein, wherein a therapeutically effective amount of solubilized progesterone and at least one solubilized estrogen drug substance, typically estradiol, are included in said formulations for the treatment of a mammal, including humans, having those disease states treated by the administration of progesterone and, menopause-related symptoms including, for example, vasomotor symptoms; in relation to treatment of hypoestrogenism related symptoms including, for example and without limitation, hot flashes and night sweats (vasomotor symptoms), sleep disturbances, mood changes and vulvo-vaginal atrophy; and osteoporosis and other non-menopausal disease states or conditions treated with supplemental estrogen (collectively, "estrogen-deficient states"). As such, the transdermal patches described herein that contain both progesterone and an estrogen drug substance can be used for the treatment of one or more progesterone-deficient states and one or more estrogen-deficient states.

In accordance with various aspects and embodiments, the solubility proportion (i.e., the proportion of a solute that enters solution) is notable. The weight ratio of estradiol to the weight of the entire solution is also notable due to the intended dose amounts, discussed herein. In particular, it is desirable to obtain a target dosage of estradiol in an amount of solution that may be readily administered via a transdermal drug delivery device.

For formulations described herein, each drug substance is administered to a mammal, including humans, in need of treatment.

An exemplary cyclic/sequential regime comprises daily delivery of from about 0.01 mg to about 2.0 mg of estradiol for 14 - 18 days, followed by daily delivery of from about 0.01 mg to about 2.0 mg of estradiol per day, and about 10 mg to about 200 mg of progesterone for 10 - 14 days. Cyclic/sequential regimes may be especially useful for menopausal females.

Other exemplary delivery dosages include one or more estrogen drug substances, typically estradiol (i.e., 17β-estradiol), for use in the formulations described herein include, without limitation, 0.01 mg, 0.025 mg, 0.05 mg, 0.0625 mg, 0.1 mg 0.125 mg, 0.25 mg, 0.375 mg, 0.50 mg, 0.625 mg, 0.75 .mg, 1.0 mg, 1.125 mg, 1.25 mg, 1.375 mg, 1.50 mg, 1.625 mg, 1.75 mg and 2.0 mg per day. In further embodiments, the daily delivery dosage estradiol is from about 0.01 mg to about 0.1 mg, from about 0.05 mg to about 0.5 mg, from about 0.1 mg to about 1.0 mg, from about 0.625 mg to about 2.0 mg, from about 0.5 mg to about 2.0 mg, from about 0.01 mg per day, and from about 0.05 mg per day.

Other exemplary delivery dosages of progesterone for use in the formulations described herein include, without limitation, 10 mg, 12.5 mg, 20 mg, 25 mg, 30 mg, 50 mg, 75 mg, 100 mg, 125 mg, 150 mg, 175 mg and 200 mg per day. In further embodiments, the daily delivery dosage of progesterone is from about 10 mg to about 100 mg, from about 20 mg to about 80 mg, from about 20 mg to about 30 mg, from about 20 mg to about 50 mg, from about 25 mg to about 75 mg, from about 100 mg to about 150 mg, from about 100 mg to about 125 mg, from about 125 mg to 150 mg, from about 10 mg, from about 20 mg, and from about 30 mg per day.

These dosage strengths for each of estradiol and progesterone can be administered in formulations described herein either alone or in combination. These dosage strengths, as used herein in transdermal delivery systems, are limited only by the drug load capacity of the respective technology. Estrogen drug substances other than estradiol may also be used in the present transdermal formulation, at the indicated label dose or as otherwise prescribed by physicians.

Accordingly, drug loads for the present transdermal pharmaceutical formulations can contain any amount necessary for partial-daily, daily, or multi-daily patches to provide the daily required doses, including the exemplary doses set forth herein.

Drug loads for patches of the present disclosure for solubilized progesterone include, for example and without limitation, about 10 mg, 12.5 mg, 20 mg, 25 mg, 30 mg, 50 mg, 75 mg, 100 mg, 125 mg, 150 mg, 175 mg and 200 mg, 250 mg, 300 mg, 350 mg, 400 mg, 450 mg, 500 mg, 550 mg, 600 mg, 650 mg, and 700 mg per patch. In further embodiments, the drug load of progesterone is not less than about 10 mg, 20 mg, 30 mg, 100 mg, 200 mg, 300 mg, 200 mg, 400 mg, and 600 mg per day.

In further embodiments, the drug load for one or more estrogen drug substances, typically estradiol (i.e., 17β-estradiol), include, for example and without limitation, about 0.01 mg, 0.025 mg, 0.05 mg, 0.0625 mg, 0.1 mg 0.125 mg, 0.25 mg, 0.375 mg, 0.50 mg, 0.625 mg, 0.75 .mg, 1.0 mg, 1.125 mg, 1.25 mg, 1.375 mg, 1.50 mg, 1.625 mg, 1.75 mg and 2.0 mg. In further embodiments the drug load for one or more estrogen drug substance is from about 0.01 mg to about 0.1 mg, from about 0.05 mg to about 0.5 mg, from about 0.1 mg to about 1.0 mg, from about 0.625 mg to about 2.0 mg, from about 0.5 mg to about 2.0 mg, from about 0.5 mg to 0.6 mg, from about 0.01 mg, and from about 0.05 mg per day.

Progesterone formulations of the present disclosure are prepared by solubilizing progesterone drug substance via blending with a pharmaceutically acceptable oil; generally, the oil comprises at least one medium chain fatty acid such as medium chain fatty acids consisting of at least one mono-, di-, or triglyceride, or derivatives thereof, or combinations thereof. Sufficient oil is used to form a solubilized progesterone. Optionally added are other excipients including, for example and without limitation, antioxidants, permeability enhancers, crystallization inhibitors and the like.

In another embodiment, a patch in accordance with the present disclosure is administered every three to four days in postmenopausal women, and may produce the following target absorption profile for estradiol and progesterone. For estradiol, a target steady-state serum concentration of about 30 to 60 pg/mL may be achieved. In another embodiment a target average steady-state serum concentration of about 45 to 50 pg/mL may be achieved, which is equivalent to the normal ranges observed at the early follicular phase in premenopausal women. These concentrations may be achieved within 12 to 24 hours following patch delivery. The patch may be configured so that minimal fluctuation in serum estradiol concentrations is achieved following patch application, which indicates a consistent hormone delivery over the application interval. For progesterone, a target average steady-state serum concentration may be achieved within 12 to 24 hours following patch delivery system.

In other aspects and embodiments, progesterone is fully solubilized using, for example and without limitation, sufficient amounts of: TRANSCUTOL (Diethylene glycol monoethyl ether); MIGLYOL (caprylic/capric triglyceride); CAPMUL PG-8 (Propylene Glycol Monocaprylate); CAPMUL PG-10 (Propylene Glycol Monocaprate); CAPMUL MCM (Medium Chain Mono- and Di-glycerides); CAPMUL MCM and a non-ionic surfactant; CAPMUL MCM and GELUCIRE (Lauroyl macrogol-32 glycerides EP Lauroyl polyoxyl-32 glycerides NF Lauroyl polyoxylglycerides (USA FDA IIG)), among others, alone or in combination.

Various ratios of these oils can be used for full solubilization of progesterone. CAPMUL MCM and a non-ionic surfactant can be used at ratios including, for example and without limitation: 65:35, 70:30, 75:25, 80:20, 85:15 and 90:10. CAPMUL MCM and GELUCIRE can be used at ratios including, for example and without limitation, 6:4, 7:3, 8:2, and 9:1. Among other combinations, these oils and/or solubilizers, as defined herein, and combinations thereof, can be used to form combination estradiol and progesterone formulations of the present disclosure. In accordance with various embodiments, formulations do not include peanut oil. The lack of peanut oil obviates the risk posed to those having peanut-based allergies.

Pharmaceutically acceptable oils include, without limitation, the use of at least one of caproic fatty acid; caprylic fatty acid; capric fatty acid; tauric acid; myristic acid; linoleic acid; succinic acid; glycerin; mono-, di-, or triglycerides and combinations and derivatives thereof; a polyethylene glycol; a polyethylene glycol glyceride (Gelucire®; GATTEFOSSE SAS, Saint-Priest, France); a propylene glycol; a caprylic/capric triglyceride (MIGLYOL®; SASOL Germany GMBH, Hamburg; MIGLYOL includes MIGLYOL 810 (Caprylic / Capric Triglyceride), MIGLYOL 812 (Caprylic / Capric Triglyceride), MIGLYOL 816 (Caprylic / Capric Triglyceride)and MIGLYOL 829 (Caprylic / Capric/Succinic Triglyceride)); a caproic/caprylic/capric/lauric triglyceride; a caprylic/capric/linoleic triglyceride; a caprylic/capric/succinic triglyceride; propylene glycol monocaprylate; propylene glycol monocaprate; (Capmul® PG-8 and 10; the CAPMUL brands are owned by ABITEC, Columbus Ohio); propylene glycol dicaprylate; propylene glycol dicaprylate; medium chain mono- and di-glycerides (CAPMUL MCM); a diethylene glycol mono ester (including 2-(2-Ethoxyethoxy)ethanol: TRANSCUTOL (Diethylene glycol monoethyl ether)); diethylene glycol monoethyl ether; esters of saturated coconut and palm kernel oil and derivatives thereof; triglycerides of fractionated vegetable fatty acids, and combinations and derivatives thereof.

In other aspects and embodiments, progesterone is fully solubilized using, for example and without limitation, sufficient amounts of: TRANSCUTOL and MIGLYOL; TRANSCUTOL, MIGLYOL and CAPMUL PG 8 and/or PG 10; CAPMUL MCM; CAPMUL MCM and a non-ionic surfactant; and CAPMUL MCM and GELUCIRE.

Various ratios of these oils can be used for full solubilization of progesterone. CAPMUL MCM and a non-ionic surfactant, e.g., GELUCIRE 44/14, can be used at ratios of about 99:1 to 2:1, including, for example and without limitation: 60:40, 65:35, 70:30, 75:25, 80:10, 80:15, 85:20, 90:10, and 98:1. The ratios of oil (e.g., medium chain fatty acid esters of monoglycerides and diglycerides) to non-ionic surfactant can be significantly higher. For example, in certain examples, below, CAPMUL MCM and GELUCIRE were used in ratios of up to about 65:1, e.g., 8:1, 22:1, 49:1, 65:1 and 66:1. See, e.g., Tables 13-17, below. Thus, useful ratios can be 8:1 or greater, e.g., 60 to 70:1. Among other combinations, these oils and/or solubilizers, as defined herein, and combinations thereof, can be used to form combination estradiol and progesterone formulations of the present disclosure.

In illustrative embodiments of the invention, oils used to solubilize estradiol and to suspend, partially solubilize, or fully solubilize progesterone include medium chain fatty acid esters, (e.g., esters of glycerol, polyethylene glycol, or propylene glycol) and mixtures thereof. In illustrative embodiments, the medium chain fatty acids are C6 to C14 or C6 to C12 fatty acids. In illustrative embodiments, the medium chain fatty acids ore saturated, or predominantly saturated, e.g., greater than about 60% or greater than about 75% saturated. In illustrative embodiments, estradiol or progesterone (or both) is soluble in the oils at room temperature, although it may be desirable to warm the oils up until they are in a liquid state. In illustrative embodiments, the oil or oil/surfactant is liquid at between room temperature and about 50C, e.g., at or below 50C, at or below 40C, or at or below 50 C. In illustrative embodiments, GELUCIRE 44/14 is heated to about 65 C and CAPMUL MCM is heated to about 40 C to facilitate mixing of the oil and non-surfactant, although such heating is not necessary to dissolve the estradiol or progesterone. In illustrative embodiments, the solubility of estradiol in the oil (or oil/surfactant) is at least about 0.5 wt%, e.g., 0.8 wt% or higher, or 1.0 wt% or higher. However, much higher solubility can be achieved. For example, as shown in the examples, below, estradiol is stable in solution on CAPMUL MCM at 12 mg/g (which is approximately equal to 12 mg/ml). As shown in Example 17, such solubility is favored over results observed in longer chain and unsaturated fatty acid esters.

Illustrative examples of mono- and diglycerides of medium chain fatty acids include, among others, CAPMUL MCM, CAPMUL MCM C10, CAPMUL MCM C8, and CAPMUL MCM C8 EP. These oils are C8 and C10 fatty acid mono- and diglycerides. Illustrative examples of oils that are triglycerides of medium chain fatty acids include, among others, MIGLYOL 810 and MIGLYOL 812.

Illustrative examples of oils that are medium chain fatty acid esters of propylene glycol include, among others, CAPMUL PG-8, CAPMUL PG-2L EP/NF, CAPMUL PG-8 NF, CAPMUL PG-12 EP/NF and Capryol. Other illustrative examples include MIGLYOL 840.

Illustrative examples of oils that are medium chain fatty acid esters of polyethylene glycol include, among others, GELUCIRE 44/14 (PEG-32 glyceryl laurate EP), which is polyethylene glycol glycerides composed of mono-, di- and triglycerides and mono- and diesters of polyethylene glycol. Without intending to be bound to any particular mechanism, it appears that at least in formulations comprising small amounts of GELUCIRE, e.g., 10 wt% or less, the primary function of this oil is as a non-ionic surfactant.

These illustrative examples comprise predominantly medium chain length, saturated, fatty acids, specifically predominantly C8 to C12 saturated fatty acids. It will be understood that commercially available fatty acid esters of glycerol and other glycols are often prepared from natural oils and therefore may comprise components additional to the fatty acid esters that comprise the predominant (by weight) component(s) and that therefore are used to characterize the product. Such other components may be, e.g., other fatty acid triglycerides, mono- and diesters, free glycerol, or free fatty acids. So, for example, when an oil/solubilizing agent is described herein as a saturated C8 fatty acid mono- or diester of glycerol, it will be understood that the predominant component of the oil, i.e., >50 wt% (e.g., >75 wt%, >85 wt% or >90 wt%) are caprylic monoglycerides and caprylic diglycerides. For example, the Technical Data Sheet by ABITEC for CAPMUL MCM C8 describes CAPMUL MCM C8 as being composed of mono and diglycerides of medium chain fatty acids (mainly caprylic) and describes the alkyl content as <= 1% C6, >= 95% C8, <= 5% C10, and <= 1.5% C12 and higher.

By way of further example, MIGLYOL 812 is generally described as a C8-C10 triglyceride because the fatty acid composition is at least about 80% caprylic (C8) acid and capric (C10) acid. However, it can also comprise small amounts of other fatty acids, e.g., less than about 5% of caproic (C6) acid, lauric (C12) acid, and myristic (C14) acid.

Specifically, a product information sheet for MIGLYOL by SASOL provides the composition of fatty acids as follows:

| Tests | 810 | 812 | 818 | 829 | 840 |
|---|---|---|---|---|---|
| Caproic acid (C6:0) | max. 2,0 | max. 2,0 | max. 2 | max. 2 | max. 2 |
| Caprylic acid (C8:0) | 65,0 - 80,0 | 50,0 - 65,0 | 45 - 65 | 45 - 55 | 65 - 80 |
| Capric acid (C10:0) | 20,0 - 35,0 | 30,0 - 45,0 | 30-45 | 30 - 40 | 20 - 35 |
| Lauric acid (C12:0) | max. 2 | max. 2 | max. 3 | max. 3 | max. 2 |
| Myristic acid (C14:0) | max. 1,0 | max. 1,0 | max. 1 | max. 1 | max. 1 |
| Linoleic acid (C18:2) | - | - | 2 - 5 | - | - |
| Succinic acid | - | - | - | 15 - 20 | - |

Where certain embodiments of this invention are described as comprising (or consisting essentially of) a capsule shell, estradiol solubilized in C8-C10 triglycerides, and a thickening agent, it will be understood that the fatty acid esters component of the formulation may be, e.g., MIGLYOL 812 or a similar product.

By way of further illustration, GELUCIRE 44/14 is generally described as lauroyl polyoxyl-32 glycerides, i.e., polyoxyethylene 32 lauric glycerides (which is a mixture of mono-, di-, and triesters of glycerol and mono- and diesters of PEGs) because the fatty acid composition is 30 to 50 % lauric acid and smaller amounts of other fatty acids, e.g., up to 15 % caprylic acid, up to 12% capric acid, up to 25% myristic acid, up to 25% palmitic acid, and up to 35% stearic acid. The product may also contain small amounts of non-esterified glycols. Where certain embodiments of this invention are described as described as comprising (or consisting essentially of) a capsule shell, estradiol solubilized in triglycerides, and a thickening agent that is a non-ionic surfactant comprising C8 to C18 fatty acid esters of glycerol and polyethylene glycol, it will be understood that the thickening agent component of the formulation may be, e.g., GELUCIRE 44/14 or a similar product.

Similarly, where certain embodiments of this invention are as described as comprising (or consisting essentially of) a capsule shell, estradiol solubilized in triglycerides, and a thickening agent that is a non-ionic surfactant comprising PEG-6 stearate, ethylene glycol palmitostearate, and PEG-32 stearate, it will be understood that the thickening agent component of the formulation may be, e.g., TEFOSE 63 or a similar product.

Mixtures of medium chain fatty acid glycerides, e.g., C6-C12, C8-C12, or C8-C10 fatty acid mono- and diglycerides or mono-, di-, and triglycerides are very well suited for dissolving estradiol; good results have been obtained with an oil that is predominantly a mixture of C8-C10 saturated fatty acid mono- and diglycerides. Longer chain glycerides appear to be not as well suited for dissolution of estradiol. On the other hand, high solubility of progesterone has been obtained in mixtures that are predominantly medium chain fatty acid triglycerides.

High solubility of estradiol has been obtained in 2-(2-Ethoxyethoxy)ethanol, e.g., TRANSCUTOL and in Propylene glycol monocaprylate, e.g., Capryol™ 90 (Gattefosse).

In illustrative embodiments of the invention, the selected oil does not require excessive heating in order to solubilize progesterone or estradiol. For example, when the formulation comprises medium chain fatty acid mono- and diglycerides (e.g., CAPMUL MCM) and polyethylene glycol glycerides (e.g., GELUCIRE) as a surfactant, the oil and/or the surfactant can be warmed up, e.g., to about 65 C in the case of the surfactant and less in the case of the oil, to facilitate mixing of the oil and surfactant. The estradiol can be added at this temperature or at lower temperatures as the mixture cools or even after it has cooled as temperatures above room temperature, e.g., about 20 C, are not required to solubilize the estradiol in preferred oils. The progesterone can also be added as the mixture cools, e.g., to below about 40 C or to below about 30 C, even down to room temperature.

In various embodiments, estradiol is solubilized. Solubilized estradiol may include estradiol that is approximately: 90% soluble in a solvent; 93% soluble in a solvent; 95% soluble in a solvent; 97% soluble in a solvent; 99% soluble in a solvent; and 100% soluble in a solvent. Solubility may be expressed as a mass fraction (% w/w, also referred to as wt%).

In addition to the oils referenced above for progesterone, which can also be used as solubilizers for estradiol, other solubilizers include, for example and without limitation, glyceryl mono- and di-caprylates, propylene glycol and 1,2,3-propanetriol (glycerol, glycerin, glycerine).

Progesterone and estrogen, in particular estradiol, may be micronized to facilitate solubilization. Progesterone and estrogen may be micronized by any one of the multiple methods typically utilized by one skilled in the art. In various embodiments, micronized progesterone has an X50 particle size value of less than about 15 microns, less than about 10 microns, less than about 5 microns and/or less than about 3 microns. In various embodiments, micronized progesterone has an X90 particle size value of less than about 25 microns, less than about 20 microns, and/or less than about 15 microns. In various embodiments, micronized estradiol has an X90 particle size value of less than about 10 microns, less than about 8 microns, and/or less than about 7 microns.

Particle size may be determined in any suitable manner. For example, a Beckman Coulter LS 13 320 Laser Diffraction Particle Size Analyzer (the "Beckman Device") may be used to determine particle size. As described above, particle size may be represented by various metrics, for example, through an X50 particle size, and/or X90 particle size, or any similar descriptions of particle size.

In various embodiments, at least one estrogen drug substance, typically estradiol, is solubilized and then combined with solubilized progesterone, or may be co-solubilized with progesterone as taught herein.

In various embodiments, the solubilizing agent for an estrogen drug substance, typically estradiol, is selected from at least one of a solvent or co-solvent. Suitable solvents and co-solvents include any mono-, di- or triglyceride and glycols, and combinations thereof. More specifically, other solubilizers include, for example and without limitation, glyceryl mono- and di-caprylates, propylene glycol and 1,2,3-propanetriol (glycerol, glycerin, glycerine).

Anionic and/or non-ionic surfactants can be used in other embodiments of the presently disclosed formulations containing solubilized progesterone or a combination of such progesterone and at least one solubilized estrogen drug substance. In certain embodiments, a non-ionic surfactant is used. Exemplary non-ionic surfactants may include, for example and without limitation, one or more of oleic acid, linoleic acid, palmitic acid, and stearic acid esters or alcohols. In further embodiments, the non-ionic surfactant may comprise polyethylene sorbitol esters, including polysorbate 80, which is commercially available under the trademark TWEEN 80® (Sigma Aldrich, St. Louis, MO). Polysorbate 80 comprises approximately 60%-70% oleic acid with the remainder comprising primarily linoleic acids, palmitic acids, and stearic acids. Polysorbate 80 may be used in amounts ranging from about 5 to 50%, and in certain embodiments, about 30% of the formulation total mass.

In various other embodiments, the non-ionic surfactant is selected from one or more of glycerol and polyethylene glycol esters of fatty acids, for example, lauroyl macrogol-32 glycerides and/or lauroyl polyoxyl-32 glycerides, commercially available as GELUCIRE, including, for example, GELUCIRE 44/11 and 44/14. These surfactants may be used at concentrations greater than about 0.01%, and, in some embodiments in amounts of about 0.01%-10.0%, 10.1%-20%, and 20.1%-30% may be used. In certain examples, below, GELUCIRE 44/14 is used as a surfactant in amounts of 1 to 10 wt%. See, e.g., Tables 13-17, below. Other non-ionic surfactants include, e.g., Labrasol® PEG-8 Caprylic/Capric Glycerides (Gattefosse) and Labarafil® corn/apricot oil PEG-6 esters (Gattefosse).

Other exemplary non-ionic surfactants include PEG-6 palmitostearate and ethylene glycol palmitostearate, which is available commercially as TEFOSE 63 (Mixture of PEG-6 stearate JPE (and) ethylene glycol palmitostearate EP/NF/JPE (and) PEG-32 stearate JPE polyoxyl 6 and polyoxyl 32 palmitostearate / glycol stearate (USA FDA IIG))("Tefose 63")(GATTEFOSSE SAS, Saint-Priest, France) which can be used with, for example, CAPMUL MCM having ratios of MCM to TEFOSE 63 of, for example, 8:2 and 9:1. Additional examples of solubilizing agents with non-ionic surfactants include, for example, MIGLYOL:GELUCIRE 50/13 (Stearoyl macrogol-32 glycerides EP Stearoyl polyoxyl-32 glycerides NF Stearoyl polyoxylglycerides (USA FDA IIG)) and MIGLYOL:TEFOSE 63.

Anionic surfactants are well known and can include, for example and without limitation: ammonium lauryl sulfate, dioctyl sodium sulfosuccinate, perfluoro-octane sulfonic acid, potassium lauryl sulfate and sodium stearate.

As such, non-ionic and/or anionic surfactants can be used alone or with at least one solubilizing agent or can be used in combination with other surfactants. Accordingly, such surfactants, or any other excipient as set forth herein, should be used to provide a solubilized progesterone drug substance; to provide a solubilized estrogen drug substance; or optionally a solubilized progesterone drug substance with at least one estrogen drug substance; upon release from a transdermal patch of the present disclosure, with consistency of dissolution and/or delivery of the solubilized progesterone drug substance and solubilized estrogen drug substance, or optionally a solubilized progesterone drug substance with estrogen drug substance, that promotes transdermal absorption.

Illustrative embodiments in which the carrier is a medium fatty acid ester of a glycol and which comprise a non-ionic surfactant as described herein are in liquid form, i.e., not gels, hard fats or other solid forms.

In additional embodiments, an antioxidant is used. Any suitable anti-oxidant may be used such as, for example and without limitation butylated hydroxytoluene.

Permeability enhancers are chemical substances that increase the flux of a drug substance through the skin. These substances may include, for example, various glycols such as dipropylene or propylene glycol, oils such as olive oil, squalene or lanolin, long chain ethers and esters, urea and various urea derivatives, solvents such as dimethylsulfoxide (DMSO) and dimethylformamide (DMF), salicylic acid, various amino acids, Vitamin-C, Vitamin-A, and Vitamin-E, and the like.

As is with all oils, solubilizers, excipients, antioxidants, permeability enhancers, crystallization inhibitors and any other additives used in intermediate formulations (e.g., during production during solublization) and during production of the resulting transdermal drug products described herein, each is to pharmaceutically acceptable.

The embodiments herein provide for the preparation and use of a solubilized progesterone transdermal patch. Such patch can be used by itself or as concomitant administration with an estrogen drug substance which can be administered via various dosage forms and via various routes of administration. Such patches can also co-administer solubilized progesterone and at least one solubilized estrogen drug substance.

Transdermal patches according to the present disclosure will vary by patch surface area, as dependent upon the dosage concentration required over the desired time for release, the actual release mechanism, and shape. Transdermal patch surface area can range, for example and without limitation, from less than about 10 cm2 to greater than about 100 cm2. For example, a transdermal patch may be from about 2.0, 2.5 cm2, 3.0 cm2, 3.5 cm2, 4.0 cm2, 4.5 cm2, 5.0 cm2, 5.5 cm2, 6.0 cm2, 6.5 cm2, 7.0 cm2, 7.5 cm2, 8.0 cm2, 8.5 cm2, 9.0 cm2, 9.5 cm2, or about 10 cm2. In various embodiments, a patch may be from about 9 cm2 to about 16 cm2 in surface area.

The transdermal patches of the present disclosure can include any conventional form such as, for example, adhesive matrix, polymeric matrix, reservoir patch, matrix or monolithic-type laminated structure, or other release-rate modifying mechanisms known in the art, and are generally comprised of one or more backing layers, adhesives, permeation enhancers, an optional rate controlling membrane and a release liner which is removed to expose the adhesives prior to application. Polymeric matrix patches also comprise a polymeric-matrix forming material.

Various delivery systems can be used to administer the formulations of the present disclosure, including, for example, encapsulation in liposomes, microbubbles, emulsions, microparticles, microcapsules, nanoparticles, microneedles and the like.

According to additional embodiments described herein, a 28-day or monthly regime of transdermal patches can be packaged in a single kit having administration days identified to improve compliance and reduce associated symptoms, among others. One or more of the transdermal patches may contain no estradiol, for example, and/or no progesterone. Transdermal patches that comprise no estrogen or progesterone drug substance may be referred to as placebos.

Transdermally administered formulations of the present disclosure containing solubilized progesterone are used for the treatment of endometrial hyperplasia, secondary amenorrhea and other disease states treated with supplemental progesterone. Generally, progesterone-containing formulations described herein are used to treat the effects of the administration of supplemental estrogen whether administered alone or in combination with solubilized estradiol of the present disclosure or other estrogen-containing formulations.

Transdermally administered formulations of the present disclosure containing solubilized estradiol are used to treat estrogen-deficient states, including vasomotor symptoms, for example, in relation to treatment of hypoestrogenism related symptoms including, for example and without limitation, hot flashes and night sweats (vasomotor symptoms), sleep disturbances, mood changes, vulvo-vaginal atrophy, and osteoporosis and other non-menopausal disease states treated with supplemental estrogen.

Various objects of the disclosure is to provide increased patient compliance secondary to ease of use in a single product kit having both progesterone and estradiol, and to reduce the metabolic and vascular side effects of the commonly used synthetic progestins in combination HRT (norethindrone acetate, medroxyprogesterone acetate, etc.) including, for example, stroke, heart attacks, blood clots and breast cancer.

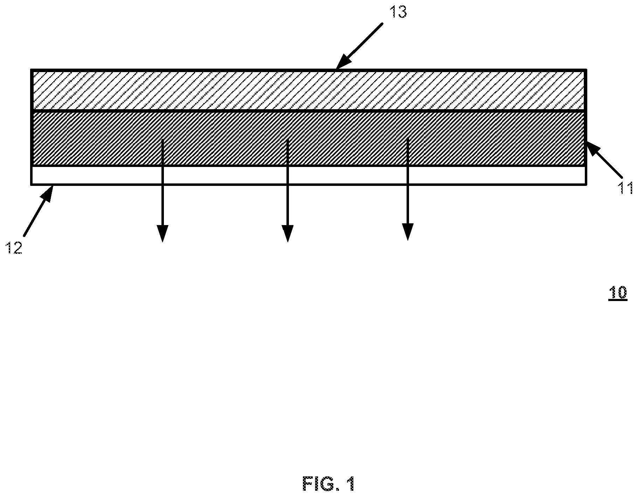

FIG. 1 illustrates the cross section of a patch 10 comprising a backing layer 13, an adhesive matrix containing active hormone(s) 11, and a removal release layer 12. The arrows in FIG.1 indicate the direction of migration of drug substance(s).

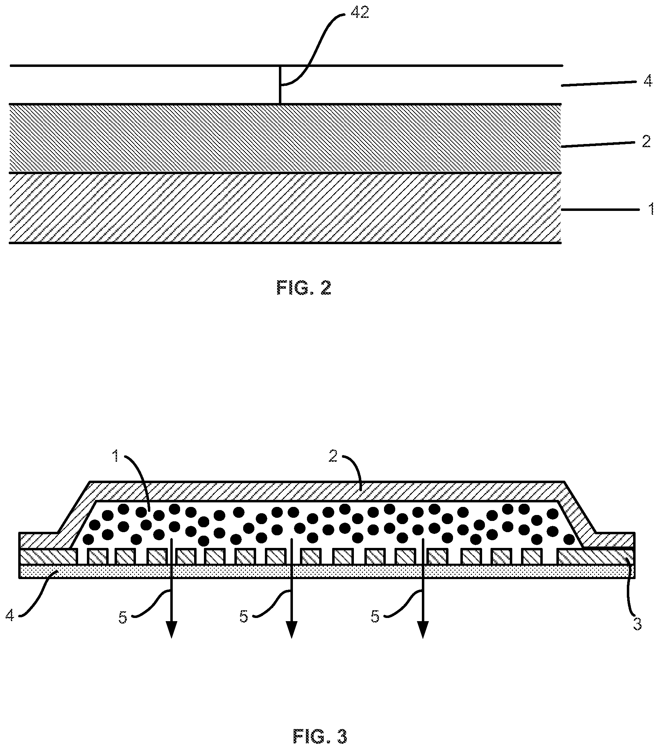

FIG. 2 illustrates another embodiment of a patch comprising a release layer 4 breakable across perforation or slit 42, and active adhesive layers 1 and 2.

FIG. 3 illustrates the cross-sectional view of an embodiment of a liquid reservoir-type transdermal delivery device. The device comprises housing 2, a porous layer 3, a removable sealing film 4, and a liquid composition 1 containing the active hormone(s). The arrows 5 in FIG. 3 illustrate the direction of migration of the drug substance(s).

In various embodiments, systems can be worn for a one-week duration while others can be worn for a 3-4 day duration (e.g., approximately 2 systems per week). Still others can be configured to be worn for other durations of time that can be shorter than 3-4 days (e.g. 1 day), or longer than one week (e.g., 2-4 weeks).

According to various embodiments, a plurality of transdermal systems can be packaged together, whether cyclic/sequential or continuous-combined. The package may comprise a packaging system further configured to guide the application of the transdermal systems throughout the intended cycle; such as throughout the application days and/or weeks within an intended dosing cycle ("sequence control device"). Such sequence control devices offer improved patient compliance and reduce associated symptoms, among other advantages. By way of example, according to various embodiments, a 28-day or monthly regime of transdermal systems can be packaged in a sequence control device having application days and/or weeks identified. According to other embodiments, a 28-day or monthly regime of transdermal systems can be packaged in a plurality of sequence control devices, for example, 2 or 4.

Various sequence control devices may be used with transdermal systems to construct kits. Exemplary sequence control devices comprise a plurality of storage elements, wherein each storage element corresponds to one or more days and wherein each storage element encloses one or more transdermal systems therein for application during the corresponding days (e.g., on the first day of the corresponding days). A storage element may comprise a blister, a laminate, or another air-tight configuration, for enclosing one or more transdermal systems therein.

Exemplary sequence control devices can optionally also comprise a plurality of spacer elements arranged between storage elements, wherein each spacer element corresponds to one or more days during which no new application of a transdermal system is prescribed. A spacer element may correspond to a placebo transdermal system or the absence of any transdermal system. Spacer elements may be especially useful for embodiments wherein one storage element corresponds to one day, and the enclosed transdermal system(s) is/are configured to be worn for more than one day.

Exemplary sequence control devices can comprise storage elements, (and optional spacer elements) sequentially coupled to a common substrate and arranged thereon to be adjacent in rows and/or columns.

Additionally, or alternatively, exemplary sequence control devices can comprise storage elements (and optional spacer elements) sequentially stacked. The sequence of such stacked elements can be maintained in various ways, for example, each of the stacked elements being coupled at one of its edges to a common substrate, or the stacked elements being contained within a container having a dispensing edge configured to only be opened large enough to dispense a single storage or spacer element at a time. In such embodiments, the stacked elements are biased toward the dispensing edge, for example, mechanically or simply under the influence of gravity if the dispensing edge is situated at the bottom of the sequence control device.

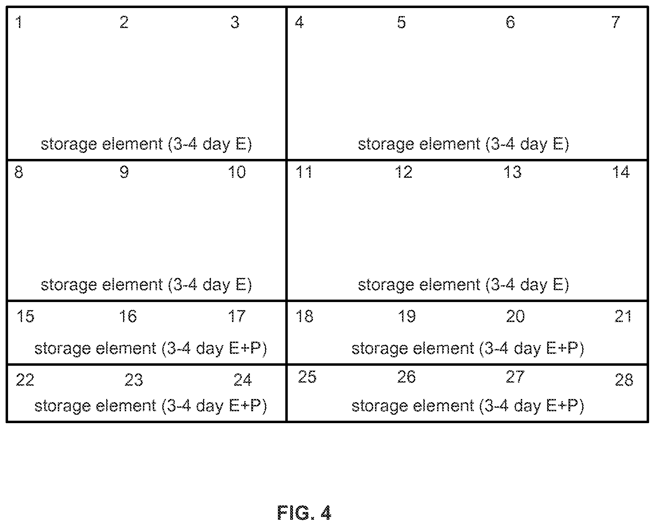

For example, FIG. 4 illustrates a 28-day cyclic/sequential estrogen ("E") progesterone ("P") regime of transdermal systems packaged in a sequence control device, wherein storage elements are sequentially arranged on a common substrate to be variously adjacent in rows and columns. The substrate can comprise a plurality of scores, perforations or dividers between adjacent elements. In this embodiment, the transdermal systems are configured to be worn for 3-4 day duration and each storage element corresponds to 3-4 days as applicable.

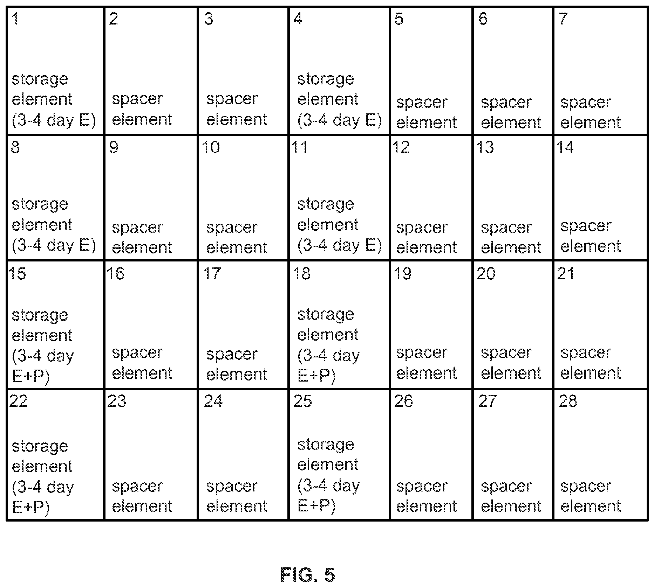

Similar to FIG. 4, FIG. 5 illustrates another 28-day cyclic/sequential estrogen ("E") progesterone ("P") regime of transdermal systems packaged in a sequence control device, wherein storage and spacer elements are sequentially arranged on a common substrate to be variously adjacent in rows and columns. As above, the substrate can comprise a plurality of scores, perforations or dividers between adjacent elements. In this embodiment, the transdermal systems are configured to be worn for 3-4 day duration and each storage element corresponds to one day.

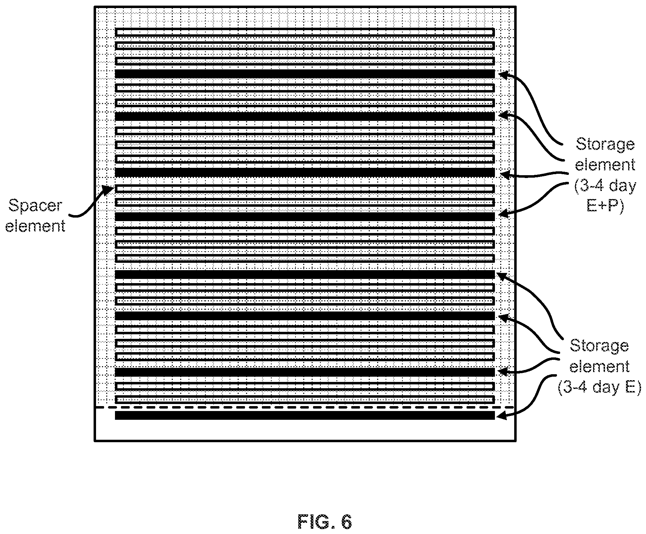

FIG. 6 illustrates another embodiment of a 28-day cyclic/sequential estrogen ("E") progesterone ("P") regime of transdermal systems packaged in a sequence control device, wherein storage and spacer elements are sequentially stacked, the stacked elements being contained within a container having a dispensing edge configured to only be opened at the bottom large enough to dispense a single storage or spacer element at a time. In this embodiment, the stacked elements are biased toward the dispensing edge under the influence of gravity. As above, in this embodiment, the transdermal systems are configured to be worn for 3-4 day duration and each storage element corresponds to one day.

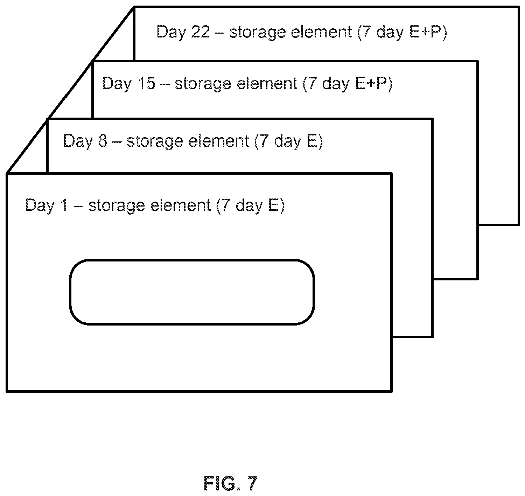

FIG. 7 illustrates another embodiment of a 28-day cyclic/sequential estrogen ("E") progesterone ("P") regime of transdermal systems packaged in a sequence control device, wherein storage elements are sequentially stacked, each of the stacked elements being coupled at one of its edges to a common substrate. In this embodiment, the transdermal systems are configured to be worn for a one-week duration and each storage element corresponds to one day.

In the case of topical creams, gels, ointments or the like, metered applicators can be used to contain and to dispense appropriate amounts of the formulation onto the skin. Certain such devices can be used to spread the formulation over an appropriate size area of the skin or the user can spread the formulation by hand or with an applicator such as an impermeable flexible film.

For example, a multi-dose metered pump dispenser is presently commercially available for topical administration of testosterone 1% and 1.62% under the brand name, AndroGel(R).

Unit dose packages can also be employed such as are also used to contain and dispense ANDROGEL.

### EXAMPLES

The following examples illustrate formulations of solubilized progesterone and estradiol, compositions of adhesive, liquid matrix compositions, and reservoir technologies, that incorporate solubilized progesterone and estradiol, for transdermal HRT, particularly NHRT. The examples illustrate target loading of adhesive compositions onto release layers of various size for the preparation of transdermal patches. The examples further illustrate exemplary loading of liquid compositions of solubilized progesterone, optionally including estradiol, into reservoirs usable for liquid transdermal delivery systems. The flux of progesterone and estradiol through the skin delivered from any of the matrices described herein can be in a variety of ranges; in one embodiment in a range of from about 0.1 to about 20 µg/cm2/hr. The flux depends on the type of matrix, i.e. adhesive or liquid compositions, the non-active ingredients, the inclusion of permeation enhancers, release-rate modifying mechanisms, crystallization inhibitors and the physiology of the mammal, typically human, subject.

### EXAMPLE 1 - Solubilized Progesterone Master Batches with Optional Estradiol

TABLE A illustrates exemplary solubilized progesterone formulations. Exemplary formulas are prepared by heating various solubilizers and then adding micronized progesterone and optionally estradiol to the heated mixture. For example, CAPMUL MCM is heated to between 30°C to 50°C, more preferably from 35°C to 45°C, and more preferably to 40°C +/- 2°C. GELUCIRE 44/14 is added to the CAPMUL MCM and mixed until dissolved. The addition is performed all at once or may be divided in portions over a period of time. Heat is continuously applied during the mixing of the requisite solubilizers. Heating and mixing are performed under an inert or relatively inert gas atmosphere. In this example, nitrogen gas (N2) is used. Mixing and/or heating is performed in any suitable vessel, such as a stainless steel vessel equipped with any suitable submerged mixer and optionally a vacuum may or may not be applied. Alternatively, GELUCIRE 44/14 is heated to 65 C and CAPMUL MCM is heated to 40 C +/-5 C to achieve mixing of the oil and the surfactant before heat is removed; estradiol is added while the mixture is cooling; progesterone is added when the mixture has dropped below about 40 C; the mixture is then passed through a colloid mill, e.g., three times.

**TABLE A: Solubilized Progesterone and Estradiol Formulations**

| **Ingredient (%w/w)** | **1** | **2** | **3** |
|---|---|---|---|
| Progesterone, USP, micronized | 7.14 | 7.34 | 9.50 |
| 17β-Estradiol hemihydrate, USP | 0.29 | 1.2 | 1.2 |
| Monoglycerides/ diglycerides/ triglycerides of caprylic and capric acid (e.g. CAPMUL® MCM, NF) | 82.57 | 91.46 | - |
| Lauroyl polyoxy-32-glycerides (e.g. GELUCIRE® 44/14, NF) | 10.0 | - | - |
| Propylene glycol monocaprylate (e.g. CAPMUL® PG8) | - | - | 89.30 |
| Total | 100 | 100 | 100 |

### EXAMPLE 2- Patch Adhesive Delivery Matrices

TABLE B illustrates adhesive matrices that are prepared by mixing appropriate polymers and then slowly mixing in the solubilized progesterone formulae from TABLE A. Volatile solvents, such as silicone fluids, low molecular weight alcohols, glycols or ethers, are added to adjust the viscosity of the adhesive composition and the tackiness of the dried adhesive. Any additional drug substance, such as estradiol, is be added to the mixture. The resulting adhesive mixture containing the drug substances are used to produce various transdermal products.

Out of the suitable polymers, the preferred polymers for a pressure sensitive adhesive (PSA) matrix, with solubilized estradiol and progesterone, include polysiloxane PSAs from Dow Corning Corporation (Midland, MI), polyacrylate, polyisobutylene, polyvinylpyrrolidone, and polyethylene/vinyl acetate co-polymer. A suitable adhesive matrix is formed from a medical silicone PSA without the need for additional polymers.

**TABLE B: Adhesive Matrices**

| **Ingredient (% w/w)** | **1** | **2** | **3** |
|---|---|---|---|
| Solubilized progesterone/estradiol (formula # from Table A) | 50(1) | 75(2) | 75(3) |
| Polyacrylate | 0-30 | 0-30 | 0-30 |
| Polysiloxane | 20-70 | 20-70 | 20-70 |
| Polyisobutylene | 0-45 | 0-45 | 0-45 |
| Polyvinylpyrrolidone | 0-20 | 0-20 | 0-20 |
| Polyethylene/vinyl acetate co-polymer | 0-40 | 0-40 | 0-40 |
| Volatile solvent (e.g. silicone fluid) | q.s. | q.s. | q.s. |
| Total | 100 | 100 | 100 |
| % w/w progesterone in delivery matrix | 3.57 | 5.505 | 7.125 |
| % w/w estradiol in delivery matrix | 0.145 | 0.900 | 0.900 |

It is noted that compositions of TABLE A will have estradiol previously added, so no additional estradiol is added into the adhesive matrix above.

### EXAMPLE 3 - Transdermal Patches

TABLE C illustrates transdermal delivery systems based on adhesive matrices exemplified in TABLE B. To prepare these exemplary transdermal patches, an exemplary composition from TABLE B is applied onto a release liner. The resulting product is dried if necessary to remove any volatiles. Lastly, a suitable backing material is applied such that the adhesive matrix is sandwiched between a release liner and a backing material. Suitable release liners are available from Dow Chemical Co. (Plaquemine, LA) and 3M (St. Paul, MN). Suitable backing material may comprise plastic films of polyethylene/, vinyl acetate resins, ethylene/vinyl acetate copolymers, polyvinyl chloride, polyurethane, and the like, or metal foils, nonwoven fabric, cloth, and various laminated films.

Resulting patch systems deliver progesterone alone, a combination of progesterone and estradiol, or estradiol alone.

**TABLE C: Transdermal Patches**

| **Matrix Dimensions** | | **Matrix (from TABLE B)** | **Matrix weight per patch (mg)** | **Theoretical drug substance(s) per patch (mg)** | |
|---|---|---|---|---|---|
| **Area (cm²)** | **Thickness (mm)** | | | **Progesterone** | **Estradiol** |
| 2.5 | 1 | Matrix 1 | 250 | 8.925 | 0.3625 |
| 2.5 | 2 | Matrix 3 | 500 | 35.625 | 4.50 |
| 5 | 1 | Matrix 1 | 500 | 17.85 | 0.725 |
| 5 | 2 | Matrix 2 | 1000 | 55.05 | 9.00 |
| 7 | 1 | Matrix 2 | 700 | 38.535 | 6.3 |
| 10 | 1 | Matrix 3 | 1000 | 71.25 | 9.00 |
| 16 | 1 | Matrix 3 | 1600 | 114.00 | 14.40 |

### EXAMPLE 4 - Liquid Transdermal Delivery Matrix

TABLE D illustrates exemplary compositions usable in liquid transdermal reservoirs. The exemplified compositions incorporate the solubilized progesterone and estradiol formulations of TABLE A as indicated. One or more saccharides are used, such as methyl cellulose, ethyl cellulose, carboxymethylcellulose, carboxymethylethylcellulose, starch, carrageenan, and pectin. The compositions further include gums such as xanthan or guar, low molecular weight glycols, and preservatives.

The examples of TABLE D can be incorporated in delivery systems such as the embodiment generalized in FIG. 3.

**TABLE D: Liquid Reservoir Compositions**

| **Ingredient (% w/w)** | **1** | **2** | **3** |
|---|---|---|---|
| Solubilized progesterone/estradiol (formula # from Table A) | 50(1) | 50(2) | 50(3) |
| Saccharide (e.g. methyl cellulose, etc.) | 20-80 | 20-80 | 20-80 |
| Propylene glycol | 0-20 | 0-20 | 0-20 |
| Water | q.s. | q.s. | q.s. |
| Total | 100 | 100 | 100 |
| % w/w progesterone in liquid composition | 3.57 | 3.67 | 4.75 |
| % w/w estradiol in liquid composition | 0.145 | 0.60 | 0.60 |

### EXAMPLE 5 - Particle Size Analysis

A particle size analysis for micronized estradiol was conducted by using a Beckman Coulter LS 13 320 Laser Diffraction Particle Size Analyzer (the "Beckman Device"). The Beckman Device uses laser diffraction and polarization intensity differential scattering (PIDS) technology to determine the particle size distribution of materials with an overall sizing range of 0.04 µm to 2000 µm in a single scan with no extrapolation. The analysis yields that a sample has an X50 value of 2.652 microns and an X90 value of 6.031 microns.

Approximately 0.01 g of a sample was dispersed with Coulter IB dispersant then diluted with about 10 ml of deionized water. The sample dilution was then sonicated for 15 seconds and added to the Beckman Device to analyze for 90 seconds. The particle size distribution was determined using the Fraunhofer optical model.

### EXAMPLE 6 - Particle Size Analysis

A particle size analysis for micronized progesterone was conducted also using the Beckman Device. A sample was prepared and analyzed. The Beckman Device particle sensor yields that the sample has an X50 of 6.67 micron, an X75 of 14.78 microns, and an X25 of 2.193 microns.

### EXAMPLE 7 - Cream

A vehicle system for use in an ointment or cream may be prepared in accordance with Table E, below. The vehicle systems of Table E have viscosity and physical states that are particularly suited for use in an ointment or cream.

**TABLE 4**

| # | **Vehicle system** | **Ratio** | **Physical state @ RT** | **Physical state @ 37°C after ∼30 minutes** | **Viscosity cps** | **Melting Time @ 37°C** | **Dispersion in water 37°C** |
|---|---|---|---|---|---|---|---|
| 8 | MCM:50/13 | 7:3 | Semisolid | Semisolid | | | |
| 9 | MCM:TEFOSE 63 | 9:1 | Semisolid | Liquid/cloudy | 150@ 25°C | Start: 1 min | Uniformly cloudy |
| | | | | | | Finish: 5 min | dispersion |
| 10 | MCM:TEFOSE 63 | 8:2 | Semisolid | Semisolid | 240@ 25°C | | Uniformly cloudy dispersion |
| 11 | MCM:TEFOSE 63 | 7:3 | Semisolid | Semisolid | 380@ 25°C | Semisolid after 30 min at 37°C, doesn't melt at 41°C either | Uniformly cloudy dispersion |
| 12 | MIGLYOL 812: 50/13 | 9:1 | Semisolid | Semisolid | 140@ 25°C | | 2 phases, oil on top |

## Claims

1. A transdermal pharmaceutical formulation comprising progesterone, wherein the progesterone is solubilized in medium chain fatty acid esters, and wherein the progesterone is present in a transdermal patch; the pharmaceutical formulation being:
(i) 7.14% w/w progesterone, 0.29% w/w 17-β estradiol, hemihydrate 82.57% w/w monoglycerides/diglycerides/triglycerides of caprylic and capric acid (e.g., Capmul MCM), and 10.0% w/w lauroyl polyoxy-32-glycerides (e.g., Gelucire 44/14);
(ii) 7.34% w/w progesterone, 1.2% w/w 17-β estradiol, hemihydrate and 91.46% w/w monoglycerides/diglycerides/triglycerides of caprylic and capric acid (e.g., Capmul MCM); or
(iii) 9.50% w/w progesterone, 1.2% w/w 17-β estradiol, hemihydrate and 89.30% w/w propylene glycol monocaprylate (e.g., Capmul PG8).

2. The transdermal pharmaceutical formulation according to claim 1, the pharmaceutical formulation being 7.14% w/w progesterone, 0.29% w/w 17-β estradiol, hemihydrate 82.57% w/w Capmul MCM, and 10.0% w/w Gelucire 44/14.

3. The transdermal pharmaceutical formulation according to claim 1, the pharmaceutical formulation being 7.34% w/w progesterone, 1.2% w/w 17-β estradiol, hemihydrate and 91.46% w/w Capmul MCM.

4. The transdermal pharmaceutical formulation according to claim 1, the pharmaceutical formulation being 9.50% w/w progesterone, 1.2% w/w 17-β estradiol, hemihydrate and 89.30% w/w Capmul PG8.

5. The transdermal pharmaceutical formulation according to any one of claims 1 to 5, wherein the progesterone is fully solubilized in the medium chain fatty acid esters.

6. The transdermal pharmaceutical formulation according to any one of claims 1 to 5, wherein the progesterone is partially solubilized in the medium chain fatty acid esters.

7. The transdermal pharmaceutical formulation according to claim 6, further comprising micronized progesterone, wherein the micronized progesterone is suspended in the medium chain fatty acid esters.

8. The transdermal pharmaceutical formulation according to any one of claims 1 to 7, wherein the 17β-estradiol is solubilized.

9. The transdermal pharmaceutical formulation according to any one of claims 1-8 for use in the treatment of a progesterone-deficient state in a mammal.

10. The transdermal pharmaceutical formulation for use according to claim 9, wherein said treatment is treatment of endometrial hyperplasia, secondary amenorrhea, or preterm birth when said mammal has a shortened cervix.

11. The transdermal pharmaceutical formulation for use according to claim 9 or claim 10, wherein said transdermal pharmaceutical formulation is the transdermal pharmaceutical formulation of any one of claims 1-8 and wherein said treatment is treatment of the progesterone-deficient state and an estrogen-deficient state.

12. The transdermal pharmaceutical formulation for use according to claim 11, wherein said treatment of an estrogen-deficient state is treatment of menopausal symptoms, hypoestrogenism symptoms, or osteoporosis.

13. The transdermal pharmaceutical formulation for use according to claim 11 or claim 12, wherein the progesterone and the 17β-estradiol are for administration in a cyclic-sequential method.

14. The transdermal pharmaceutical formulation for use according to claim 11 or claim 12, wherein the progesterone and the 17β-estradiol are for administration in a continuous-combined method.

## Patentansprüche

1. Eine transdermale pharmazeutische Formulierung, beinhaltend Progesteron, wobei das Progesteron in mittelkettigen Fettsäureestern solubilisiert ist und wobei das Progesteron in einem transdermalen Pflaster vorhanden ist; wobei die pharmazeutische Formulierung Folgendes ist:
(i) 7,14 Gew.-% Progesteron, 0,29 Gew.-% 17-β-Estradiolhemihydrat, 82,57 Gew.-% Monoglyceride/Diglyceride/Triglyceride von Capryl- und Caprinsäure (z. B. Capmul MCM) und 10,0 Gew.-% Lauroyl-Polyoxy-32-glyceride (z. B. Gelucire 44/14);
(ii) 7,34 Gew.-% Progesteron, 1,2 Gew.-% 17-β-Estradiolhemihydrat und 91,46 Gew.-% Monoglyceride/Diglyceride/Triglyceride von Capryl- und Caprinsäure (z. B. Capmul MCM); oder
(iii) 9,50 Gew.-% Progesteron, 1,2 Gew.-% 17-β-Estradiolhemihydrat und 89,30 Gew.-% Propylenglykolmonocaprylat (z. B. Capmul PG8).

2. Transdermale pharmazeutische Formulierung gemäß Anspruch 1, wobei die pharmazeutische Formulierung 7,14 Gew.-% Progesteron, 0,29 Gew.-% 17-ß-Estradiolhemihydrat, 82,57 Gew.-% Capmul MCM und 10,0 Gew.-% Gelucire 44/14 ist.

3. Transdermale pharmazeutische Formulierung gemäß Anspruch 1, wobei die pharmazeutische Formulierung 7,34 Gew.-% Progesteron, 1,2 Gew.-% 17-ß-Estradiolhemihydrat und 91,46 Gew.-% Capmul MCM ist.

4. Transdermale pharmazeutische Formulierung gemäß Anspruch 1, wobei die pharmazeutische Formulierung 9,50 Gew.-% Progesteron, 1,2 Gew.-% 17-ß-Estradiolhemihydrat und 89,30 Gew.-% Capmul PG8 ist.

5. Transdermale pharmazeutische Formulierung gemäß einem der Ansprüche 1 bis 5, wobei das Progesteron in den mittelkettigen Fettsäureestern vollständig solubilisiert ist.

6. Transdermale pharmazeutische Formulierung gemäß einem der Ansprüche 1 bis 5, wobei das Progesteron in den mittelkettigen Fettsäureestern teilweise solubilisiert ist.

7. Transdermale pharmazeutische Formulierung gemäß Anspruch 6, ferner beinhaltend mikronisiertes Progesteron, wobei das mikronisierte Progesteron in den mittelkettigen Fettsäureestern suspendiert ist.

8. Transdermale pharmazeutische Formulierung gemäß einem der Ansprüche 1 bis 7, wobei das 17-β-Estradiol solubilisiert ist.

9. Transdermale pharmazeutische Formulierung gemäß einem der Ansprüche 1-8 zur Verwendung bei der Behandlung eines Progesteron-Mangelzustands bei einem Säugetier.

10. Transdermale pharmazeutische Formulierung zur Verwendung gemäß Anspruch 9, wobei die Behandlung die Behandlung von Endometriumhyperplasie, sekundärer Amenorrhoe oder Frühgeburt ist, wenn das Säugetier einen verkürzten Gebärmutterhals aufweist.

11. Transdermale pharmazeutische Formulierung zur Verwendung gemäß Anspruch 9 oder Anspruch 10, wobei die transdermale pharmazeutische Formulierung die transdermale pharmazeutische Formulierung gemäß einem der Ansprüche 1-8 ist und wobei die Behandlung die Behandlung des Progesteron-Mangelzustands und eines Östrogen-Mangelzustands ist.

12. Transdermale pharmazeutische Formulierung zur Verwendung gemäß Anspruch 11, wobei die Behandlung eines Östrogen-Mangelzustands die Behandlung von Menopausesymptomen, Hypoöstrogenismussymptomen oder Osteoporose ist.

13. Transdermale pharmazeutische Formulierung zur Verwendung gemäß Anspruch 11 oder Anspruch 12, wobei das Progesteron und das 17-β-Estradiol zur Verabreichung in einem zyklisch-sequentiellen Verfahren vorliegen.

14. Transdermale pharmazeutische Formulierung zur Verwendung gemäß Anspruch 11 oder Anspruch 12, wobei das Progesteron und das 17-β-Estradiol zur Verabreichung in einem kontinuierlich kombinierten Verfahren vorliegen.

## Revendications

1. Une préparation pharmaceutique transdermique comprenant de la progestérone, dans laquelle la progestérone est solubilisée dans des esters d'acides gras à chaîne moyenne, et dans laquelle la progestérone est présente dans un timbre transdermique ; la préparation pharmaceutique étant :
(i) 7,14 % p/p de progestérone, 0,29 % p/p de 17-β estradiol hémihydrate, 82,57 % p/p de monoglycérides/diglycérides/triglycérides d'acide caprylique et caprique (p. ex., Capmul MCM), et 10,0 % p/p de glycérides de lauroyl polyoxy-32 (p. ex., Gelucire 44/14) ;
(ii) 7,34 % p/p de progestérone, 1,2 % p/p de 17-β estradiol hémihydrate et 91,46 % p/p de monoglycérides/diglycérides/triglycérides d'acide caprylique et caprique (p. ex., Capmul MCM) ; ou
(iii) 9,50 % p/p de progestérone, 1,2 % p/p de 17-β estradiol hémihydrate et 89,30 % p/p de propylène glycol monocaprylate (p. ex., Capmul PG8).

2. La préparation pharmaceutique transdermique selon la revendication 1, la préparation pharmaceutique étant 7,14 % p/p de progestérone, 0,29 % p/p de 17-β estradiol hémihydrate, 82,57 % p/p de Capmul MCM, et 10,0 % p/p de Gelucire 44/14.

3. La préparation pharmaceutique transdermique selon la revendication 1, la préparation pharmaceutique étant 7,34 % p/p de progestérone, 1,2 % p/p de 17-β estradiol hémihydrate et 91,46 % p/p de Capmul MCM.

4. La préparation pharmaceutique transdermique selon la revendication 1, la préparation pharmaceutique étant 9,50 % p/p de progestérone, 1,2 % p/p de 17-β estradiol hémihydrate et 89,30 % p/p de Capmul PG8.

5. La préparation pharmaceutique transdermique selon l'une quelconque des revendications 1 à 5, dans laquelle la progestérone est entièrement solubilisée dans les esters d'acides gras à chaîne moyenne.

6. La préparation pharmaceutique transdermique selon l'une quelconque des revendications 1 à 5, dans laquelle la progestérone est partiellement solubilisée dans les esters d'acides gras à chaîne moyenne.

7. La préparation pharmaceutique transdermique selon la revendication 6, comprenant en sus de la progestérone micronisée, dans laquelle la progestérone micronisée est mise en suspension dans les esters d'acides gras à chaîne moyenne.

8. La préparation pharmaceutique transdermique selon l'une quelconque des revendications 1 à 7, dans laquelle le 17-β estradiol est solubilisé.

9. La préparation pharmaceutique transdermique selon l'une quelconque des revendications 1 à 8 pour une utilisation dans le traitement d'un état lié à une insuffisance en progestérone chez un mammifère.

10. La préparation pharmaceutique transdermique pour une utilisation selon la revendication 9, dans laquelle ledit traitement est un traitement de l'hyperplasie endométriale, de l'aménorrhée secondaire ou de l'accouchement prématuré lorsque ledit mammifère a un col raccourci.

11. La préparation pharmaceutique transdermique pour une utilisation selon la revendication 9 ou la revendication 10, dans laquelle ladite préparation pharmaceutique transdermique est la préparation pharmaceutique transdermique de l'une quelconque des revendications 1 à 8 et dans laquelle ledit traitement est le traitement de l'état lié à une insuffisance en progestérone et d'un état lié à une insuffisance en œstrogènes.

12. La préparation pharmaceutique transdermique pour une utilisation selon la revendication 11, dans laquelle ledit traitement d'un état lié à une insuffisance en œstrogènes est le traitement de symptômes de la ménopause, de symptômes de l'hypoestrogénie, ou de l'ostéoporose.

13. La préparation pharmaceutique transdermique pour une utilisation selon la revendication 11 ou la revendication 12, dans laquelle la progestérone et le 17-β estradiol sont destinés à une administration selon une méthode séquentielle-cyclique.

14. La préparation pharmaceutique transdermique pour une utilisation selon la revendication 11 ou la revendication 12, dans laquelle la progestérone et le 17-β estradiol sont destinés à une administration selon une méthode combinée-continue.
